# EUROPÄISCHE PATENTSCHRIFT

(11) **EP 2 515 660 B1**
(45) Veröffentlichungstag und Bekanntmachung des Hinweises auf die Patenterteilung: **25.02.2015**
(21) Anmeldenummer: 10805411.5
(22) Anmeldetag: 21.12.2010
(51) Int. Cl.: A01N 25/04, A01N 25/22, A01N 25/34, A01N 59/16, B82Y 40/00, B82Y 30/00, C09C 1/62

(54) **FORMULIERUNG MIT SILBERNANOPARTIKEL**
FORMULATIONS WITH SILVER NANOPARTICLES
FORMULATIONS AVEC DES NANOPARTICULES D'ARGENT

(30) Priorität: 22.12.2009 DE 102009059276
(43) Veröffentlichungstag der Anmeldung: 31.10.2012
(73) Patentinhaber: Rent-a-Scientist GmbH, 93059 Regensburg (DE)
(72) Erfinder: NUSKO, Robert, 93109 Wiesent (DE); MAIER, Georg, 93177 Altenthann (DE)
(74) Vertreter: Graf Glück Kritzenberger
(86) Internationale Anmeldenummer: PCT/DE2010/075165
(87) Internationale Veröffentlichungsnummer: WO 2011/076203

(56) Entgegenhaltungen:
- EP-A1- 2 050 792
- WO-A2-2007/082155
- CN-A- 101 296 744
- JP-A- 2008 037 949
- JP-A- 2008 231 489
- KR-A- 20080 077 613
- ZHANG X ET AL: "Effects of various polyoxyethylene sorbitan monooils (Tweens) and sodium dodecyl sulfate on reflux synthesis of copper nanoparticles", MATERIALS RESEARCH BULLETIN, ELSEVIER, KIDLINGTON, GB, Bd. 41, Nr. 11, 9. November 2006 (2006-11-09), Seiten 2041-2048, XP025249226, ISSN: 0025-5408, DOI: DOI:10.1016/J.MATERRESBULL.2006.04.008 [gefunden am 2006-11-09]

## Beschreibung

### Technisches Gebiet

Die Erfindung betrifft eine silbernanopartikelhaltige, disperse, wässrige Formulierung sowie Verfahren zu deren Herstellung.

### Stand der Technik

Einer allgemeinen Definition folgend ist "Nanopartikel" eine Bezeichnung für Partikel, die eine Größe im Bereich kleiner als 100 nm aufweisen. Die Verwendung der Vorsilbe "Nano" stellt somit, entsprechend der offiziellen Definition nach ISO TC 229, eine Abgrenzung zu Partikel im Sub-Mikrometer Bereich (> 100 nm) dar. Generell ist davon auszugehen, dass Stoffe, die als Nanomaterial bezeichnet werden, veränderte chemische und physikalische Eigenschaften besitzen. Bei Nanometallen zeigen z.B. Gold und Metall andere Farben als die entsprechenden Metalle, nämlich Rot bzw. Gelb.

Darüber hinaus ist es wissenschaftlich belegt, dass Nanopartikel eines Stoffes eine erhöhte Oberflächenenergie besitzen. Je kleiner die Partikel sind desto höher ist deren Oberflächenenergie. In der Konsequenz sind Nanopartikel generell als instabil zu betrachten, da sie aufgrund ihrer hohen Oberflächenenergie leicht zu neuen Verbindungen bzw. größeren, stabileren Aggregaten reagieren. Für das Beispiel der Nanometalle bedeutet dies, dass selbst die Partikel edler Metalle schnell mit Luftsauerstoff oxidieren sobald die Größe der Partikel im Bereich von Nanometern liegt.

Technologisch nutzbare Nanopartikel erhält man also nur, wenn deren Oberflächen chemisch oder physikalisch geschützt und damit stabilisiert sind. Als "technologisch nutzbar" können Nanopartikel bezeichnet werden, die von der Herstellung über die Verarbeitung bis hin zur Anwendung unverändert ihre ursprüngliche Partikelgröße behalten oder konservieren.

Möglichkeiten der Stabilisierung von Nanopartikeln in Dispersionen sind aus dem Stand der Technik bekannt. Es existieren drei wesentliche Verfahrenswege zur Herstellung von metallischen Nanopartikeln. In einem ersten Verfahren werden die Nanopartikel zur Stabilisierung auf Feststoffen geträgert. Die Feststoffe liegen dabei immer in einer stabilen Größe im Mikrometerbereich vor. Auf diese Weise hergestellte Materialien sind in WO 2008/017176 A1 und WO 2009/072911 A1 beschrieben. Nachteilig bei der Verwendung der auf diese Weise hergestellten Produkte ist einerseits der Verlust der Nanoskaligkeit und andereseits die hohe Füllstofffracht. Der verwendete Füllstoff, der als Basis für die Entstehung der Metallnanopartikel dient, weist Korngrößen im Bereich von Mikrometern auf und ist z.B. für die Herstellung von dünnen Strukturen oder Fasern gänzlich ungeeignet. In der Praxis beträgt zudem der Gewichtsanteil des Füllstoffes ein Vielfaches des Nanometallanteiles.

Bei flammenpyrolytischen Verfahrenswegen, wie in WO 2008/017176 A1, EP 1 889 810 A1 und DE 10 2008 025 108 A1 beschrieben, fällt das Cluster bestehend aus Mikro- und Nanopartikeln als Feststoff an, der zur Weiterverwendung erst aufwändig redispergiert werden muss, was aufgrund von Lagerungseinflüssen oft nicht mehr vollständig möglich ist. Darüber hinaus kann die Verteilung der Nanopartikel niemals optimal erfolgen, da sie höchstens so gut sein kann wie die Verteilung der Mikropartikel, auf denen sie abgeschieden sind.

Ein zweiter Verfahrensweg besteht in der Synthese von Metallnanopartikel durch Stabilisierung mittels Polymeren wie Polyvinylpyrrolidon im Polyolprozess, der vielfach in der Literatur als Standardverfahren beschrieben ist. Allerdings werden hier nur geringe Metallnanopartikelkonzentrationen erreicht (Bereich kleiner 0.1 Gew.-% Metall).

Die dritte Variante zur Erzeugung von Metalinanopartikel ist ein PVD Verfahren (Physical Vapor Deposition), bei dem das zugrunde liegende Metall verdampft wird. Zur Stabilisierung der so erzeugten Nanopartikel werden wiederum Polymere bzw. Silikone verwendet. Die Erzeugung von Metalldampf ist ein sehr energieaufwändiger Prozess, der evakuierte Prozesskammern erfordert. Diese Herstellungsverfahren sind damit unökonomisch. Zudem bereiten die verwendeten Polymere und Silikone bei der Weiterverarbeitung erhebliche prozesstechnische Probleme, da eine Redispergierung oft unmöglich ist.

Daneben ist aus der DE 10 2006 056 284 A1 die Herstellung einer antimikrobiell wirkenden wässrigen Dispersion durch Mischen einer wässrigen Dispersion nanoscaliger Teilchen, die wenigstens ein antimikrobiell wirkendes Metall enthalten, mit einer wässrigen Dispersion eines Polymerisations-, Polykondensations- oder Polyadditionsproduktes bekannt. Die Herstellung der Metall-Nanopartikel erfolgt durch eine chemische Reduktion in Wasser. Zur Stabilisierung der Metall-Nanopartikel wird Natriumchlorid verwendet

Ein Nachteil, den alle Herstellungsvarianten zeigen, ist die schlechte Verarbeitbarkeit der Metallnanopartikel in Polymerschmelzen, wie etwa bei der Additivierung von thermoplastischen Polymeren. Feststoffe lassen sich ohne vorherige Dispergierung nicht homogen einarbeiten.

Es besteht daher weiterhin ein Bedarf an stabilen Dispersionen von Metallnanopartikeln und insbesondere Silbernanopartikeln mit einer großen Bandbreite an Einsatzmöglichkeiten in der nachfolgenden Verwendung.

### Darstellung der Erfindung

Hier setzt die Erfindung an. Der Erfindung, wie sie in den Ansprüchen gekennzeichnet ist, liegt die Aufgabe zugrunde, stabile Dispersionen von Silbernanopartikeln bereitzustellen, die in einer großen Zahl an Anwendungen eingesetzt werden können.

Diese Aufgabe wird erfindungsgemäß durch die silbemanopartikelhaltige Formulierung gemäß Anspruch 1 und das Verfahren zur Herstellung einer silbernanopartikelhaltigen Formulierung gemäß Anspruch 9 gelöst. Weitere vorteilhafte Details, Aspekte und Ausgestaltungen der vorliegenden Erfindung ergeben sich aus den abhängigen Ansprüchen, der Beschreibung, den Beispielen und den Figuren.

Die vorliegende Erfindung stellt eine silbernanopartikelhaltige, disperse, wässrige Formulierung umfassend 0,5 bis 60 Gew.-% Silbernanopartikel und zumindest zwei Stabilisatoren aus der Gruppe der nichtionischen Tenside, wobei das Mengenverhältnis Silbernanopartikel zu Stabilisator im Bereich von 10 : 2 bis zu 10 : 50 liegt, wobei die zumindest zwei Stabilisatoren ausgewählt sind aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester, Polyoxypropylen-tri-alkylsäureester und deren Gemische zur Verfügung.

Im vorliegenden Text beziehen sich sämtliche Angaben von Anteilen in Gew.-% auf das Gewicht der gesamten Formulierung als 100%-Basis.

Die erfindungsgemäße Formulierung enthält zumindest zwei Stabilisatoren ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester und Polyoxypropylen-tri-alkylsäureester. Bei diesen Stabilisatoren handelt es sich um Verbindungen mit grenzflächenaktiven Eigenschaften aus der Gruppe der nichtionischen Tenside, die bei Raumtemperatur in flüssiger Form vorliegen. Nichtionische Tenside im Sinne der Erfindung sind grenzflächenaktive chemische Komponenten, die ungeladene polare und unpolare Bereiche in einem Molekül aufweisen. Darüber hinaus weisen nichtionische Tenside keine dissoziierbaren funktionellen Gruppen auf.

Eine erfindungsgemäße silbernanopartikelhaltige Formulierung enthält dispersionsstabilisierte Silbernanopartikel, die nicht zu größeren Agglomeraten aggregieren können, da die verwendeten Stabilisatoren im Temperaturbereich von 0-240 °C flüssig sind. Im Gegensatz dazu hält der S tand der Technik beispielsweise viele Silbernanopartikelprodukte bereit, die als trockene Pulver angeboten werden, die aber aufgrund ihrer Agglomerationsneigung während Transport und Lagerung zur Dispergierung in organischen Lösungsmitteln, wie Acryl- und Epoxidharzen, nur unter hohem mechanischen Energieeintrag und dann auch nur unvollständig redispergiert werden können.

Bei den zumindest zwei Stabilisatoren kann es sich dabei um verschiedene Stabilisatoren einer der genannten Klassen chemischer Verbindungen handeln oder um Stabilisatoren verschiedener Verbindungsklassen. Bei einer Kombination von drei verschiedenen Stabilisatoren können also beispielsweise drei verschiedene Polyoxyethylen-mono-alkylsäureester eingesetzt werden, es können aber beispielsweise auch zwei verschiedene Polyoxyethylen-mono-alkylsäureester und ein Polyoxypropylen-mono-alkylsäureester oder beispielsweise ein Polyoxypropylen-di-alkylsäureester, ein Polyoxyethylen-tri-alkylsäureester und ein Polyoxypropylen-tri-alkylsäureester verwendet werden. Es ist jede beliebige Kombination dieser nichtionischen Tenside möglich.

Besonders bevorzugt sind in der Formulierung Stabilisatoren in Form einer Kombination nichtionischer Tenside aus zwei unterschiedlichen der oben genannten Verbindungsklassen enthalten.

Gemäß einer besonders bevorzugten Ausführungsform sind die Stabilisatoren ausgewählt aus der Gruppe bestehend aus Polyoxyethylen-Sorbitan-Monolaurat, Polyoxyethylen-Sorbitan-Monopalmitat, Polyoxyethylen-Sorbitan-Monostearat, Polyoxyethylen-Sorbitan-Monooleat, Polyoxyethylen-Sorbitan-Tristearat, Polyoxyethylen-Glyceryl-Trioleat, Polyoxyethylen-Glyceryl-Monolaurat, Polyoxyethylen-Glyceryl-Monooleat, Polyoxyethylen-Glyceryl-Monostearat, Polyoxyethylen-Glyceryl-Monoricinoleat, Rizinusöl, hydriertes Rizinusöl und Sojabohnenöl.

Die Stabilisatoren sind vielfach nicht unter ihrem Chemikaliennamen bekannt sondern unter ihrer jeweiligen Handelsbezeichnung. Im Rahmen der vorliegenden

Erfindung bevorzugte Stabilisatoren sind Tween20^{™}, Tween40^{™}, Tween60^{™}, Tween80^{™}, Polysorbat^{™}, Tagat TO^{™}, Tagat TO V^{™}, Tagat L2^{™}, Tagat S2^{™}, Tagat R40^{™}, Triton X 100^{™}, Hydrogenated Castoroil^{™}, PEG 20 Glycerylstearat ^{™}. PEG 20 Glyceryllaurat^{™}, PEG 40 Castoroil^{™}, PEG 25 Glyceryltrioleat^{™}, Newcol^{™}, Montane^{™}, Lonzest^{™}, Liposorb^{™}, Nonion^{™}, Kuplur^{™}, Ionet^{™}, Kemotan^{™}, Grillosan^{™}, Ethylan^{™}, Glycomul^{™}, Emsorb^{™}, Disponil^{™}, Amisol^{™}, Armotan^{™}, Sorbax^{™}, Sorbitan^{™}, Span^{™} und Tego Pearl^{™}.

Diese Liste von Stabilisatoren ist nicht vollständig, da verschiedene Hersteller gleiche oder ähnliche Produkte unter anderen Namen vermarkten bzw. neue nichtionische Tenside der oben genannten Verbindungsklassen in der Zukunft synthetisiert werden und ebenfalls in einer erfindungsgemäßen Mischung verwendet werden können.

Bevorzugt sind die zumindest zwei Stabilisatoren in der Formulierung in einem Mengenverhältnis im Bereich von 1 : 1 bis 2 : 1 anwesend.

Da zumindest zwei nichtionischen Tenside als Stabilisatoren für das gebildete Nanosilber verwendet werden besteht ein Mengenzusammenhang zwischen den Konzentrationen der Stabilisatoren und des Silbers. Die erfindungsgemäße Formulierung weist ein Mengenverhältnis Silber zu Stabilisatoren im Bereich von 10 : 2 bis 10 : 50 auf. Bevorzugt beträgt das Mengenverhältnis Silber zu Stabilisatoren von 10 : 5 bis 10 : 20, besonders bevorzugt von 10 : 6 bis 10 : 10. Unter dem "Mengenverhältnis Silber zu Stabilisator" ist das "Mengenverhältnis Silber zur Summe der anwesenden Stabilisatoren" zu verstehen. Bei Verwendung der bevorzugten Mengenverhältnisse erhält man besonders stabile Dispersionen von Silbernanopartikeln, die universell eingesetzt werden können.

Bevorzugt weisen die Silbernanopartikel eine Partikelgröße von 1 nm bis 100 nm, besonders bevorzugt 1 nm bis 50 nm, insbesondere bevorzugt 1 nm bis 20 nm auf.

Die erfindungsgemäße Formulierung ist flüssig und enthält ansonsten keinerlei feste Nebenbestandteile, die die Möglichkeiten der weiteren Verwendung einschränken würden.

In der erfindungsgemäßen Formulierung sind eine oder mehrere grenzflächenaktive Komponenten als Stabilisatoren enthalten, die neben der Stabilisierung der Silbernanopartikel auch die Weiterverarbeitung (durch nochmalige Dispergierung, Emulgierung) in alle anderen Substrate ermöglichen. Die technologisch anspruchsvollste Weiterverarbeitung ist dabei die Prozessierung in thermoplastischen Kunststoffen. Die hierbei angewendeten Temperaturen reichen bis 300 °C. Bis zu dieser Temperatur ist es wünschenswert, dass die zur Additivierung verwendete Formulierung flüssig ist, was durch die Verwendung eines oder mehrere grenzflächenaktiver Komponenten, die bis zu einer Temperatur von kurzzeitig 300 °C flüssig sind, erfüllt ist.

Bevorzugt sind mehr als zwei Stabilisatoren in der erfindungsgemäßen Formulierung anwesend. In diesem Fall liegt der Gehalt eines ersten Stabilisators im Bereich von 30 Gew.-% bis 90 Gew.-%, bevorzugt zwischen 40 Gew.-% bis 60 Gew.-%, besonders bevorzugt zwischen 45 Gew.-% bis 55 Gew.-%. Der verbleibende Gewichtsanteil bis 100 Prozent teilt sich unter die weiteren in Kombination verwendeten erfindungsgemäßen Stabilisatoren auf, die sich ihrerseits in Mengenanteilen von 0 bis 100 Gew.-% aufteilen. Die hier genannten Gew.-%-Angaben beziehen sich somit in Abweichung zu sonstigen Angaben auf das Gesamtgewicht an Stabilisatoren als 100%-Basis.

Besonders bevorzugt sind in der Formulierung ein oder mehrere Stabilisatoren ausgewählt aus der Gruppe bestehend aus Tagat TO V^{™}, Tween20^{™}, Tween80^{™} und Tagat L2^{™} anwesend. Unter Verwendung dieser Stabilisatoren werden besonders stabile und universell einsetzbare Dispersionen erhalten.

Gemäß einer ganz besonders bevorzugten Ausführungsform der vorliegenden Erfindung ist in der Formulierung als Stabilisator ein Gemisch von Tagat TO V^{™} und Tween20^{™} anwesend. Insbesondere bevorzugt sind Formulierungen, in denen das Mengenverhältnis Tagat TO V^{™} zu Tween20^{™} im Bereich von 1 : 2 bis zu 2 : 1 liegt und ganz besonders bevorzugt sind Formulierungen, in denen das Mengenverhältnis Tagat TO V^{™} zu Tween20^{™} ungefähr 1 : 1 beträgt.

Bezüglich der Partikelgröße sei nochmals auf die eingangs formulierte Definition verwiesen, wonach die Silbernanopartikel eine Partikelgröße von weniger als 100 nm aufweisen. In der erfindungsgemäßen Formulierung liegen die Silbernanopartikel in einer Partikelgröße von 1 nm bis 100 nm, bevorzugt 1 nm bis 50 nm, besonders bevorzugt 1 nm bis 20 nm vor. Die Morphologie der Silbernanoteilchen kann dabei Formen von Dreiecken, Kuben, Sphären, Stäben oder Plättchen aufweisen.

Bevorzugt enthält die Formulierung stabile, nanoskalige Silber-Partikel in einer Konzentration von 0,5 Gew.-% bis 60 Gew.-%, wobei das Mengenverhältnis Silbernanopartikel zu Stabilisator im Bereich von 10 : 5 bis zu 10 : 10 liegt. In dem bevorzugten Bereich erhält man besonders stabile Dispersionen von Silbernanopartikeln, die universell eingesetzt werden können.

Besonders bevorzugt sind die Silbernanopartikel in einem Anteil von 1 Gew.-% bis 40 Gew.-%, bevorzugt in einem Anteil von 5 Gew.-% bis 30 Gew.-% in der Formulierung enthalten.

Besonders bevorzugt enthält die silbernanopartikelhaltige, disperse, wässrige Formulierung zumindest 70 Gew.-% Wasser.

Die vorliegende Erfindung umfasst auch ein Verfahren zur Herstellung der oben beschriebenen silbernanopartikelhaltigen, dispersen, wässrigen Formulierungen umfassend die Schritte Bereitstellen eines Silbersalzes, Bereitstellen von zumindest zwei Stabilisatoren ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester, Polyoxypropylen-tri-alkylsäureester und der Gemischen, Bereitstellen eines Reduktionsmittels, Bereitstellen des Lösungsmittels Wasser, Herstellen einer Lösung von Silbersalz, Stabilisator und Reduktionsmittel, Zugabe einer Base zu der Lösung, wobei die Zugabe der Base kontinuierlich über einen Zeitraum von 5 h bis 48 h derart erfolgt, dass der pH-Wert der Formulierung zwischen 0 und 6 liegt.

Durch das erfindungsgemäße Herstellungsverfahren wird eine Formulierung mit einer sehr engen Verteilung der Partikelgrößen der Nanopartikel erhalten.

Bevorzugt erfolgt die Zugabe der Base kontinuierlich über einen Zeitraum von 9 h bis 30 h. Auf diese Weise wird eine Formulierung mit einer besonders engen Verteilung der Partikelgrößen der Nanopartikel erhalten.

Das erfindungsgemäße Herstellungsverfahren ist ein reduktiver chemischer Prozess. Demnach werden die Silberpartikel durch chemische Reduktion aus ihren Salzen hergestellt. Generell kann zur Herstellung der erfindungsgemäßen Silbernanopartikel jedes beliebige chemische oder physikalische Reduktionsmittel verwendet werden. Unter physikalischen Reduktionsmitteln ist hier Temperaturerhöhung oder Bestrahlung mit Licht zu verstehen. Vorteilhaft ist die Verwendung eines chemischen Reduktionsmittels, da hier Stoffumsätze von 100 Prozent und sehr hohe Reaktionsgeschwindigkeiten erzielt werden können.

Überraschenderweise hat sich gezeigt, dass bei Anwesenheit von zumindest zwei Stabilisatoren aus der Gruppe Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester und Polyoxypropylen-tri-alkylsäureester sehr starke Reduktionsmittel verwendet werden können, was zu einer erhöhten Reaktionsgeschwindigkeit führt, ohne gleichzeitige Gefahr der Bildung eines größeren Anteils großer, unerwünschter Silberpartikel.

Unter den chemischen Reduktionsmitteln sind solche bevorzugt, die keine in der Reaktionsmischung verbleibenden Reaktionsnebenprodukte, wie etwa die entsprechende oxidierte Form des jeweiligen Reduktionsmittel, erzeugen, welche die Qualität der silbernanopartikelhaltigen, dispersen, wässrigen Formulierung mindern würden. Gemäß einer bevorzugten Ausführungsform wird daher ein Reduktionsmittel eingesetzt, das mit den Silberionen des Silbersalzes unter Bildung von elementarem Silber und ansonsten überwiegend gasförmigen Reaktionsprodukten reagiert.

Besonders bevorzugt werden Reduktionsmittel, die in ihrer oxidierten Form als gasförmiger Stoff die Reaktionslösung verlassen können, wie etwa Hydrazinhydrat. Selbstverständlich können die erfindungsgemäßen Silbernanopartikel ebenso mit jedem beliebigen anderen Reduktionsmittel erhalten werden.

Zur Herstellung der erfindungsgemäßen Formulierung können beliebige Silbersalze verwendet werden. Aus Gründen der Praktikabilität hängt die Wahl des Silbersalzes von dem eingesetzten Lösungsmittel ab, da unterschiedliche Salze unterschiedliche Lösungseigenschaften besitzen. Bevorzugt wird immer dasjenige Silbersalz, das im jeweiligen Lösungsmittel die beste Löslichkeit besitzt. In den nachfolgenden Beispielen sind einige Lösungsmittel und das jeweilig geeignete Silbersalz beschrieben. Das erfindungsgemäße Verfahren ist aber nicht auf die genannten Beispiele von Silbersalz/Lösungsmittel-Paarungen beschränkt.

Die reduktive Herstellung von Silber kann als ein Paar von Redox-Gleichungen formuliert werden. Die erste Teilgleichung ist die Reduktionsgleichung, nach der das Silberkation aus dem Silbersalz zum Elementsilber reduziert wird. Die zweite Teilgleichung beschreibt den entsprechenden oxidativen Vorgang für die Oxidation des Reduktionsmittel zum korrespondierenden Oxidationsprodukt, das idealerweise in gasförmigem Zustand die Reaktionslösung verlässt. Allen Reduktionsmitteln ist gemeinsam, dass je übertragenem Elektron ein Proton entsteht. Dieses Proton trägt dazu bei, dass der pH-Wert der gesamten Reaktionslösung sehr stark fällt. Die Abnahme des pH-Wertes ist dafür verantwortlich, dass die Reaktion unerwünschterweise zum Erliegen kommt. Deshalb muss eine Lauge zugegeben werden, um die entstehenden Protonen, die die Gesamtreaktion bremsen, abzufangen.

Überraschenderweise wurde gefunden, dass die Art der Lauge, die Konzentration der Lauge und die Geschwindigkeit, mit der die Lauge zugegeben wird, entscheidend ist für die Verteilung der Partikelgrößen der Nanopartikel in der hergestellten Formulierung.

Bevorzugt wird als Base Ammoniak, Kaliumhydrogencarbonat oder Natriumhydroxid eingesetzt. Bei Verwendung dieser Basen werden besonders stabile Dispersionen mit einer engen Verteilung der Partikelgrößen der Nanopartikel erhalten.

Die zugegebene Menge an Lauge ist dabei so zu bemessen, dass nach Abschtuss der Reaktion eine pH neutrale Dispersion erhalten wird. Der pH-Wert liegt dann zwischen pH 5 und pH 9.

Laugen bzw. Protonen-Akzeptoren sind definiert durch ihre pK_{b} Werte. Der pK_{b} Wert ist der negative dekadische Logarithmus der Protonenkonzentration im Gleichgewicht und ist somit ein Maß für die Stärke der Base.

Zur Herstellung der erfindungsgemäßen Formulierung sind Basen geeignet, die einen pK_{b} Wert im Bereich von -2 bis 10,5, bevorzugt 1,5 bis 9,1, besonders bevorzugt im Bereich 3,5 bis 7,5 aufweisen.

Neben der Basenstärke ist zudem die Zugabegeschwindigkeit in die Reaktionslösung zur Herstellung der erfindungsgemäßen Formulierung entscheidend. Erfolgt die Zugabe zu schnell, so verschiebt sich das Partikelgrößen-Spektrum hin zu größeren Partikeln, die im Extremfall im Mikrometerbereich liegen. Erfolgt dagegen die Zugabe zu langsam, so werden keine Stoffausbeuten größer 90 % erhalten, da bereits gebildetes Nanosilber katalytisch den Abbau von Reduktionsmitteln hervorruft und damit kein Reaktionspartner mehr zur Erzeugung von Silbernanopartikeln vorliegt.

Experimente haben gezeigt, dass die Zugabegeschwindigkeit der Lauge bei einer Ansatzgröße von 50 kg im Bereich von 9 bis 30 Stunden liegen sollte, um die erfindungsgemäße hohe Qualität an Silbernanopartikeln zu erreichen. Bei entsprechend geringeren Ansatzgrößen verringert sich analog auch die Zeit der Laugenzugabe. Nach oben kann die Zugabezeit nicht beliebig erweitert werden, da der katalytische Abbau des Reduktionsmittels durch bereits entstandenes Nanosilber spätestens nach 48 h die Gesamtausbeute merklich beeinträchtigt.

Die Zugabegeschwindigkeit der Lauge erfolgt so, dass der pH-Wert der Dispersion stets zwischen 0 und 6 liegt. Ein höherer pH-Wert führt zu einer zu schnellen Reaktion und damit zu unkontrolliertem Partikelwachstum. Ein zu niedriger pH-Wert führt dazu, dass die Reaktion zum Erliegen kommt und damit kein Nanosilber mehr gebildet wird.

Die erfindungsgemäße Formulierung kann in einer Vielzahl von Anwendungen zum Einsatz kommen, wobei bei den verschiedensten Verwendungen deutlich vorteilhafte Eigenschaften erreicht werden. Die Silbernanopartikel der erfindungsgemäßen Formulierung bzw. die Silbernanopartikel einer nach dem erfindungsgemäßen Verfahren hergestellten Formulierung können insbesondere zur Erzielung folgender Funktionen in verschiedene Substrate eingearbeitet werden: Antimikrobielle Aktivität, Förderung des Pflanzenwachstums, elektrische Leitfähigkeit, Antistatik, Farbigkeit, Katalyse etc.

Eine nanopartikelhaltige Mischung kann durch Zugabe eines anorganischen Salzes aus einer entsprechenden nanopartikelhaltigen Formulierung gewonnen werden. Dabei ist es aber unerheblich, ob die nanopartikelhaltige Formulierung durch Dispergieren einer nanopartikelhaltigen Mischung hergestellt wurde oder ob die nanopartikelhaltige Formulierung durch Reduktion eines Silbersalzes in Lösung gewonnen wurde.

Insbesondere bei der Herstellung einer nanopartikelhaltigen Mischung aus einer nanopartikelhaltigen Formulierung, die durch Reduktion eines Silbersalzes in Lösung gewonnen wurde, ist die Zugabe eines anorganischen Salzes mit ganz besonderen Vorteilen verbunden. Es wurde nämlich überraschenderweise gefunden, dass sich durch Zugabe anorganischer Salze die durch Zugabe einer Base zu der Lösung von Silbersalz, Stabilisatoren und Reduktionsmittel erhaltene Dispersion in zwei chemische Phasen 1 und 2 trennt. Phase 1 enthält dabei die Silbernanopartikel in dem verwendeten flüssigen Stabilisatorengemisch. Das Lösungsmittel, die anorganischen Salze und das Nebenprodukt Ammoniumnitrat befinden sich in der über der Phase 1 stehenden Phase 2. Die beiden Phasen lassen sich nunmehr auf einfache Weise voneinander separieren, indem die obere Phase 2 abdekantiert wird. Zurück bleibt die Phase 1, die nur mehr aus den SilberNanopartikeln und den flüssigen Stabilisatoren besteht.

Die in Form einer Dispersion vorliegenden Silbernanopartikel, die in der Regel Partikelgrößen von 1 nm - 20 nm aufweisen und chemisch stabilisiert sind, werden auf diese Weise von dem in der Dispersion enthaltenen Nebenprodukt Ammoniumnitrat und dem verwendeten Lösungsmittel, also insbesondere Wasser, abgetrennt. Die erfindungsgemäßen silbernanopartikulären Formulierungen können dadurch zusätzlichen Applikationsfeldern zugänglich gemacht werden. Zu diesen Applikationsfeldern gehören Anwendungen, in denen das verwendete Lösungsmittel und das Nebenprodukt Ammoniumnitrat stören. Insbesondere stehen Wasser und Ammoniumnitrat in Anwendungen mit polaren oder aprotischen Lösungsmitteln einer Dispergierung der stabilisierten Silber-Nanopartikel entgegen.

Besonders gute Ergebnisse werden erzielt, wenn es sich bei dem Lösungsmittel um Wasser und bei den anorganischen Salzen um wasserlösliche anorganische Salze handelt. In diesem Fall wird durch Zugabe wasserlöslicher anorganischer Salze die durch Zugabe einer Base zu der Lösung von Silbersalz, Stabilisator und Reduktionsmittel erhaltene Dispersion in zwei chemische Phasen 1 und 2 getrennt. Phase 1 enthält wiederum die Silbernanopartikel in dem verwendeten flüssigen Stabilisatorengemisch. Das Lösungsmittel Wasser, die wasserlöslichen Salze und das Nebenprodukt Ammoniumnitrat befinden sich in der über der Phase 1 stehenden Phase 2. Die beiden Phasen lassen sich separieren, indem die obere wässrige Phase 2 abdekantiert wird. Zurück bleibt die Phase 1, die nur mehr aus den Silber-Nanopartikeln und den flüssigen Stabilisatoren besteht.

Zur näheren Charakterisierung geeigneter Salze müssen deren kationische und anionische Bestandteile gesondert betrachtet werden. Salze bestehen generell aus mindestens einem Kation und mindestens einem Anion. Eine unerwünschte Wechselwirkung von Kationen mit der stabilisierten silbernanopartikelhaltigen Formulierung ist nicht zu erwarten, da das Silber und insbesondere das bevorzugt verwendete Silber entweder als ungeladenes Silber oder als positiv geladenes Kation vorliegt.

Da als Lösungsmittel Wasser verwendet wird, liegt der Auswahl der geeigneten Kationen/Anionen Kombinationen in Form geeigneter Salze die Vorstellung zugrunde, dass die Phasentrennung hervorgerufen wird durch die Beanspruchung der Hydratationsfähigkeit des Wassers durch geladene Ionen. Die Hydratationsfähigkeit ist dabei ausgedrückt durch die Fähigkeit Wasserstoffbrücken ausbilden zu können. Sie ist zudem der Grund für die Stabilität der Dispersion im Dispersionsmedium Wasser. Damit herrscht als eine gewisse Konkurrenz zwischen den die Silbernanopartikel umhüllenden Stabilisatoren und den gelösten Reaktionsnebenprodukten wie beispielsweise Ammoniumnitrat.

Bei den verwendeten erfindungsgemäßen Stabilisatoren handelt es sich um nichtionische Makromoleküle, die lediglich durch schwache Dipol-Dipol-Wechselwirkungen mit den Wassermolekülen in Verbindung stehen. Die Zugabe von Salzen beabsichtigt also den stabilisierenden Einfluss der Wasserstoffbrücken auf die Gesamtdispersion zu entziehen, indem elektrisch geladene Ionen, die wesentlich stärkere Wechselwirkungen mit den Wasserdipolen eingehen, eingebracht werden. In der Regel ist dabei der Grad der Wechselwirkung mit Wasser vom Ionenradius und von der lonenladung in der Form abhängig, dass die Wechselwirkung mit sinkenden lonenradien und wachsender lonenladung zunimmt. Bei der Auswahl geeigneter Salze ist auch deren Löslichkeit in Wasser von Bedeutung. Mit steigender Löslichkeit der Salze in Wasser nimmt auch die Ausbildung von Wechselwirkungskräften mit den Wassermolekülen zu.

Die Auswahl der Anionen ist zudem dadurch eingeschränkt, dass ungewünschte Wechselwirkungen mit vorhandenen Silber-Kationen vermieden werden müssen. Damit scheiden alle Anionen aus, die mit dem eingesetzten Silber und insbesondere mit Silber schwerlösliche Verbindungen bilden, also z.B. Halogenide, Chalkogenide und deren Sauerstoffverbindungen.

Besonders bevorzugt umfasst das anorganische Salz daher zumindest ein Kation der Periode 3 oder der Periode 4 des Periodensystems der Elemente und zumindest ein Element der fünften Hauptgruppe des Periodensystems der Elemente als Bestandteil des Anions, wobei insbesondere bevorzugt das anorganische Salz Stickstoff als Bestandteil des Anions umfasst.

Besonders bevorzugt erfolgt anschließend ein Abdekantieren der sich nach Zugabe des anorganischen Salzes bildenden Phase von der im wesentlichen aus Silbernanopartikel und Stabilisatoren bestehenden silbernanopartikelhaltigen Mischung.

Eine der oben näher beschriebenen silbernanopartikelhaltigen Formulierungen kann hergestelt werden durch ein Verfahren umfassend die Schritte Bereitstellen einer der silbernanopartikelhaltigen Mischung, Bereitstellen des Lösungsmittels Wasser, Zugabe der silbernanopartikelhaltigen Mischung zu dem Lösungsmittel.

Die chemisch stabilisierten Silbernanopartikel mit einer bevorzugten Partikelgröße von 1 nm - 20 nm können also unter Zuhilfenahme von Netz-und Dispergieradditiven u.a. in organischen Lösungsmitteln, insbesondere in Acryl- und Epoxidharze, eingearbeitet werden. Diese Einarbeitung kann mit einfachsten Rühr- oder Mischtechniken erfolgen, da eine Redispergierung der Silbernanopartikel durch die Verwendung der erfindungsgemäßen Stabilisatoren nicht erforderlich ist. Auf diese Weise werden stabile Dispersionen von beispielsweise Silbernanopartikeln mit einer Partikelgröße von bevorzugt kleiner 20 nm in organischen Lösungsmitteln, bevorzugt in Acryl- und Epoxidharze, in einer Konzentration von 500 mg/kg bis 5.000 mg/kg Silbergehalt, erhalten.

Die vorliegende Erfindung umfasst auch die Verwendung der erfindungsgemäßen Formulierung zur Oberflächenbehandlung von Holz. Die mit dieser Art der Verwendung erzielten besonderen Vorteile werden in den nachfolgenden Beispielen näher erläutert.

Die vorliegende Erfindung umfasst auch die Verwendung der erfindungsgemäßen Formulierung in Imprägnierölen für Luftfilter.

Die vorliegende Erfindung umfasst auch die Verwendung der erfindungsgemäßen Formulierung als textile Färbelösung. Die erfindungsgemäße Formulierung wird dabei textilen Färbebädern zugesetzt.

Die vorliegende Erfindung umfasst auch die Verwendung der erfindungsgemäßen Formulierung zur Wachstumsförderung von Pflanzen. Die mit dieser Art der Verwendung erzielten besonderen Vorteile werden in den nachfolgenden Beispielen näher erläutert.

Die vorliegende Erfindung umfasst auch die Verwendung der erfindungsgemäßen Formulierung zur Herstellung antimikrobieller Oberflächen. Die mit dieser Art der Verwendung erzielten besonderen Vorteile werden in den nachfolgenden Beispielen näher erläutert.

Die vorliegende Erfindung umfasst auch die Verwendung der erfindungsgemäßen Formulierung zur Herstellung von Beschichtungen für Vlies-/Folienlaminate. Die mit dieser Art der Verwendung erzielten besonderen Vorteile werden in den nachfolgenden Beispielen näher erläutert.

Die vorliegende Erfindung umfasst auch die Verwendung der erfindungsgemäßen Formulierung zur Herstellung von Beschichtungen für textile Dekorstoffe. Die mit dieser Art der Verwendung erzielten besonderen Vorteile werden in den nachfolgenden Beispielen näher erläutert.

Die vorliegende Erfindung umfasst auch die Verwendung der erfindungsgemäßen Formulierung in Lacken und Klebstoffen. Die mit dieser Art der Verwendung erzielten besonderen Vorteile werden in den nachfolgenden Beispielen näher erläutert.

Die vorliegende Erfindung umfasst auch die Verwendung der erfindungsgemäßen Formulierung in Silikonen. Die mit dieser Art der Verwendung erzielten besonderen Vorteile werden in den nachfolgenden Beispielen näher erläutert.

Die vorliegende Erfindung umfasst auch die Verwendung der erfindungsgemäßen Formulierung in thermoplastischen Kunststoffen, bevorzugt in Polypropylen. Die mit dieser Art der Verwendung erzielten besonderen Vorteile werden in den nachfolgenden Beispielen näher erläutert.

Die vorliegende Erfindung umfasst auch die Verwendung der erfindungsgemäßen Formulierung in Wood Plastic Composites (WPC). Die mit dieser Art der Verwendung erzielten besonderen Vorteile werden in den nachfolgenden Beispielen näher erläutert.

Die vorliegende Erfindung umfasst auch die Verwendung der erfindungsgemäßen Formulierung in Polyolefin Formkörpern. Die mit dieser Art der Verwendung erzielten besonderen Vorteile werden in den nachfolgenden Beispielen näher erläutert.

Die vorliegende Erfindung umfasst auch die Verwendung der erfindungsgemäßen Formulierung in PVC und Plastisolen, bevorzugt die Verwendung zur Herstellung von PVC Plastisol beschichteten Textilien. Die mit dieser Art der Verwendung erzielten besonderen Vorteile werden in den nachfolgenden Beispielen näher erläutert.

Die vorliegende Erfindung umfasst auch die Verwendung der erfindungsgemäßen Formulierung zur Herstellung von Fasern, insbesondere zur Herstellung von Synthesefasern und Regeneratfasern. Die mit dieser Art der Verwendung erzielten besonderen Vorteile werden in den nachfolgenden Beispielen näher erläutert.

### Kurze Beschreibung der Zeichnungen

Zur Illustration der Erfindung und zur Verdeutlichung ihrer Vorzüge werden nachfolgend Ausführungsbeispiele angegeben. Diese Ausführungsbeispiele sollen im Zusammenhang mit den Zeichnungen näher erläutert werden. Es versteht sich von selbst, dass diese Angaben die Erfindung nicht beschränken sollen. Es zeigen
- Fig. 1: ein UVvis-Spektrum einer 5.000-fach verdünnten wässrigen Lösung einer erfindungsgemäßen Formulierung;
- Fig. 2: Gemessene Partikelgrößen und berechnete Kurve der Scanning Electron Microscope (SEM)-Datensätze.
- Fig. 3: Transmission Electron Microscope (TEM) Analyse der Silber-Nanopartikel.
- Fig. 4: eine transmissionselektronenmikroskopische Aufnahme (TEM) einer 5.000-fach verdünnten wässrigen Lösung einer erfindungsgemäßen Formulierung;
- Fig. 5: eine transmissionselektronenmikroskopische Aufnahme (TEM) eines beschichteten Vlies-/Folienlaminats;
- Fig. 6: die Kinetik der Abtötung von auf das beschichtete Vlies der Figur 5 aufgebrachten Bakterien (*E.coli*.);
- Fig. 7: eine transmissionselektronenmikroskopische Aufnahme (TEM) eines Polyester Masterbatches mit 6500 mg/kg Silber;
- Fig. 8: Microfaserstränge aus PET/PA mit 200 mg/kg Nanosilber;
- Fig. 9: Elutionsverhalten und antimikrobielle Aktivität verschiedener Polyester Mikrofasern.

### Wege zur Ausführung der Erfindung

### Beispiel 1:

### Formulierung von Nanosilber mit Hydrazinhydrat, Ammoniak, Tagat TO V^{™} und Tween20

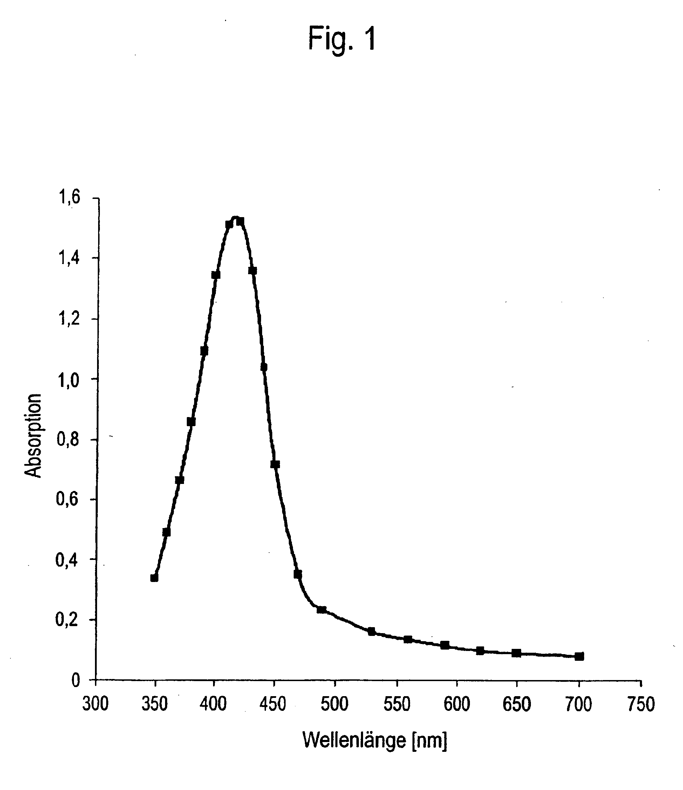

Vorgelegt werden 7.000 g Silbernitrat, 1.760 g Tagat TO V^{™}, 1.760 g Tween20^{™} und 512 g Hydrazinhydrat in 28.439 g entionisiertem Wasser. Die Lösung wird für 3 Stunden gerührt. Dann werden 5.000 g Ammoniak-Lösung (14 %-ig) über einen Zeitraum von 24 Stunden kontinuierlich zugetropft. Die Reaktion ist nach erfolgter Zugabe abgeschlossen und liefert eine Dispersion mit einem Silbergehalt von 10.0 Gew.-%. Die Partikelgröße und Verteilung wird mittels eines UVvis Spektrums (Figur 1) ermittelt. Im Ergebnis wird eine 10 prozentige Nanosilberdispersion erhalten mit einer Nanosilber-Partikelgröße von 1-30 nm.

Das Absorptionsspektrum wird an einer 5.000-fach verdünnten wässrigen Lösung durchgeführt, die 20 ppm Nanosilber enthält, klar und tiefgelb gefärbt ist. Das UVvis Spektrum wird im Wellenlängenbereich von 750 nm bis 350 nm aufgenommen. Die gemessenen Absorptionswerte liefern einen Peak mit einem Maximum bei 410 nm - 420 nm und einer Peak-Halbwertsbreite von rund 80 nm.

Die Dispergiereigenschaften der erhaltenen 10 prozentigen Dispersion sind sowohl in polaren als auch in unpolaren Lösungmittel hervorragend, das heißt es wird ohne weiteren chemischen Aufwand (Dispergierhilfen) oder mechanischen Aufwand (Ultraschall, Ultraturax etc.) eine absolut klare Lösung, die lediglich eine durch den Plasmoneffekt des Silbers verursachte Färbung aufweist, erhalten.

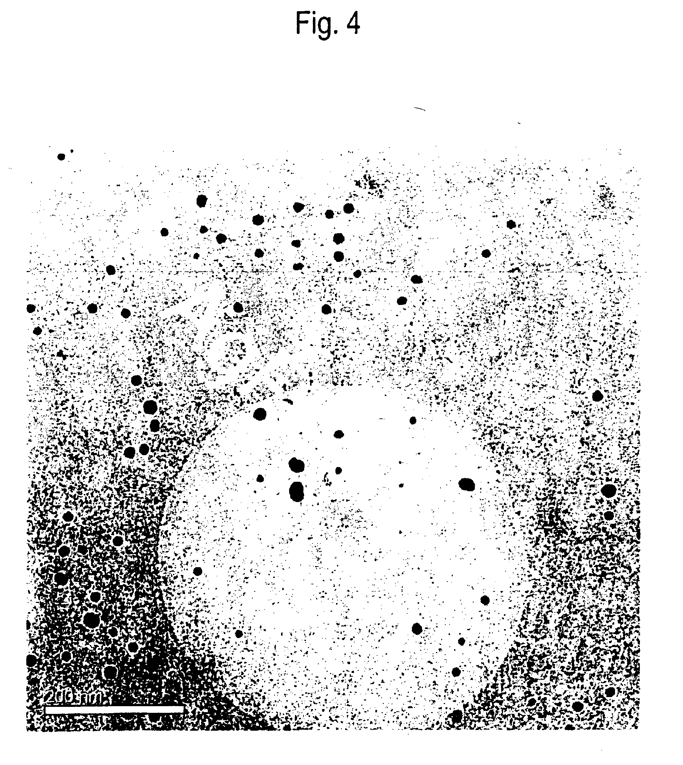

Figur 4 zeigt eine Transmissionselektronenmikroskopische Aufnahme (TEM) der verdünnten Dispersion aus Beispiel 1. Die in der Figur 4 sichtbaren dunklen Bereiche entsprechen den Nano-Silber-Partikeln, die eine Partikelgröße von 1 - 30 nm aufweisen.

### Beispiel 2:

### Formulierung von Nanosilber mit Hydrazinsulfat, Kaliumhydrogencarbonat, Tagat TO V^{™} und Tween80^{™}

Vorgelegt werden 7.000 g Silbernitrat, 2.360 g Tagat TO V^{™}, 1.160 g Tween80^{™} und 1.331 g Hydrazinsulfat in 27.620 g entionisiertem Wasser. Die Lösung wird für 3 Stunden gerührt. Dann werden 5.000 g Kaliumhydrogencarbonat-Lösung (1.900 g KHCO₃) über einen Zeitraum von 30 Stunden kontinuierlich zugetropft. Die Reaktion ist nach erfolgter Zugabe abgeschlossen und liefert eine Dispersion mit einem Silbergehalt von 10.0 Gew.-%. Die Partikelgröße und Verteilung wird mittels eines UVvis-Spektrums (Figur 1) ermittelt. Im Ergebnis wird eine 10 prozentige Nanosilberdispersion erhalten mit einer Nanosilber-Partikelgröße von 1-30 nm.

### Beispiel 3:

### Formulierung von Nanosilber mit Glucose, Natriumhydroxid, Tagat L2^{™} und Tween20^{™}

Vorgelegt werden 7.000 g Silbernitrat, 2.360 g Tagat L2^{™}, 1.160 g Tween20^{™} und 3.708 g Glucose in 25.243 g entionisiertem Wasser. Die Lösung wird für 3 Stunden gerührt. Dann werden 5.000 g Natriumhydroxid-Lösung (760 g NaOH) über einen Zeitraum von 30 Stunden kontinuierlich zugetropft. Die Reaktion ist nach erfolgter Zugabe abgeschlossen und liefert eine Dispersion mit einem Silbergehalt von 10.0 Gew.-%. Die Partikelgröße und Verteilung wird mittels eines UVvis Spektrums ermittelt. Im Ergebnis wird eine 10 prozentige Nanosilberdispersion erhalten mit einer Nanosilber-Partikelgröße von 1-30 nm.

### Beispiel 4:

### Weiterverarbeitung der Dispersion aus Beispiel 1 zur Herstellung einer flüssigen, wasser- und salzfreien Formulierung mit Silbernanopartikel

Die in Beispiel 1 erhaltene Silbernanopartikel enthaltende wässrige Dispersion ist mit dem Nebenprodukt Ammoniumnitrat verunreinigt. Die Silbernanopartikel weisen Partikelgrößen von 1-20 nm auf und sind chemisch stabilisiert. Der Gehalt an Silber beträgt 25 Gew.-%. Der Gehalt an Wasser beträgt 366 g und der Gehalt an Ammoniumnitrat 185 g. Von dieser Dispersion werden 1.000 g in ein Becherglas gegeben und unter Rühren auf 45°C geheizt. Die Tren nung der Dispersion in zwei Phasen wird durch Zugabe von 78 g Kaliumnitrat eingeleitet. Nach erfolgter Zugabe und dem vollständigen Auflösen des Kaliumnitrats wird die Heizung entfernt und der Rührer ausgeschaltet. Die Trennung der Phasen kann nach Abkühlung beobachtet werden. Die obere, klare, wässrige Phase 1 wird vollständig abdekantiert. Die zurückbleibende Phase 2 besitzt eine dunkelbraune Farbe mit sirupartiger Fließeigenschaft und einem Gewicht von 449 g. Die stabile Dispersion ist nun bereit für die Einarbeitung in ein beliebiges organisches Lösungsmittel, insbesondere in ein Acryl- oder Epoxidharz.

Eine Analyse der wässrigen Phase 1 ergibt einen Salzgehalt von 263 g und einen Wassergehalt von 366 g.

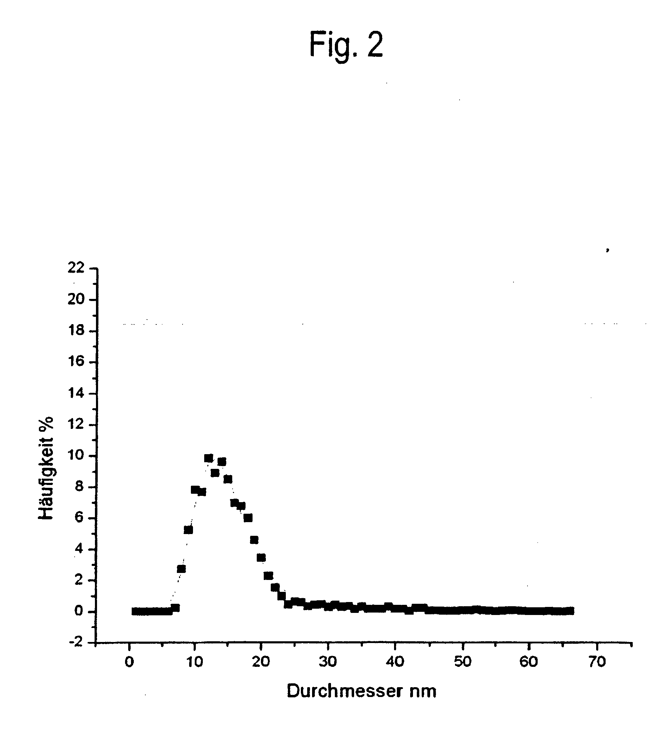

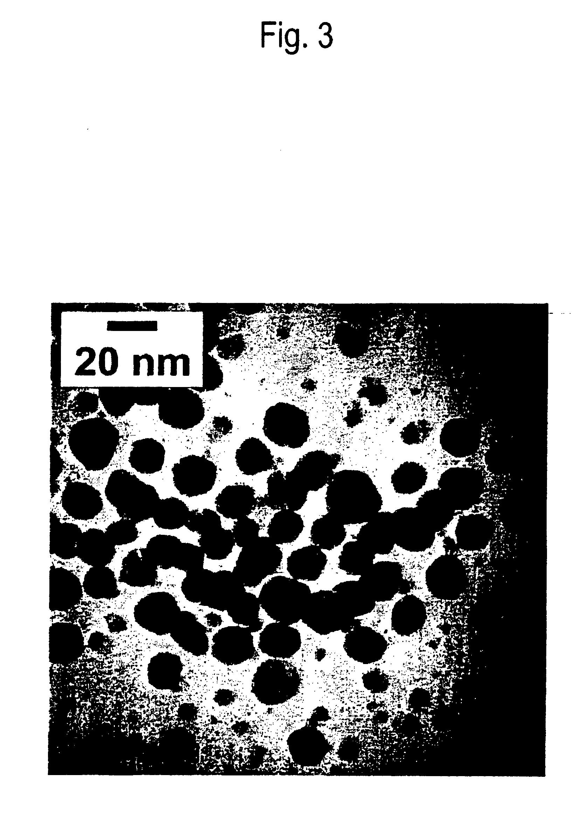

Die Analyse der silberhaltigen Phase 2 ergibt folgende Daten:
a) Die Analyse des Gesamt-Silbergehaltes durch Veraschung bei 800°C ergibt 250 g Silber. Bezogen auf die Gesamtformulierung entspricht dies einem Silbergehalt von 56 Gew-%.
b) Die Analyse der Partikelgrößenverteilung des erhaltenen Silbers ist in Figur 1 wiedergegeben. Die Bestimmung erfolgt durch Messung des UVvis-Absorptionsspektrums im Wellenlängenbereich von 700 nm - 350 nm. Das Peakmaximum bei 415 nm entspricht einer Partikelgröße von 10 nm. Die Peakhalbwertsbreite von maximal 80 nm ist ein Maß für die enge Partikelgrößenverteilung. Die Korrelation mit den SEM (Scanning Electron Microscope) und den TEM (Transmission Electron Microscope) Analysen, wie in Figur 2 und Figur 3 dargestellt, erlaubt die Angabe der Partikelgrößenverteilung von D 100 < 20 nm (100 % der Partikeldurchmesser sind kleiner als 20 nm).

### Beispiel 5:

### Verwendung der erfindungsgemäßen Formulierung zur Herstellung von Beschichtungen für Vlies-/Folienlaminate

Die Nanosilber Dispersion aus Beispiel wird in einer Konzentration von 150 mg/kg (bezogen auf den Silbergehalt in der Beschichtung) wie aus dem Stand der Technik bekannt in eine handelsübliche, zur Beschichtung von Folien bestimmte Fluorpolymerdisperion eingearbeitet. Es wird eine stabile, leicht gelb gefärbte Dispersion aus Nanosilber Partikeln und Fluorpolymerpartikeln erhalten. Diese Beschichtung wird auf ein PP-Spinnvlies/Folienlaminat aufgerakelt und thermisch getrocknet/fixiert/vernetzt.

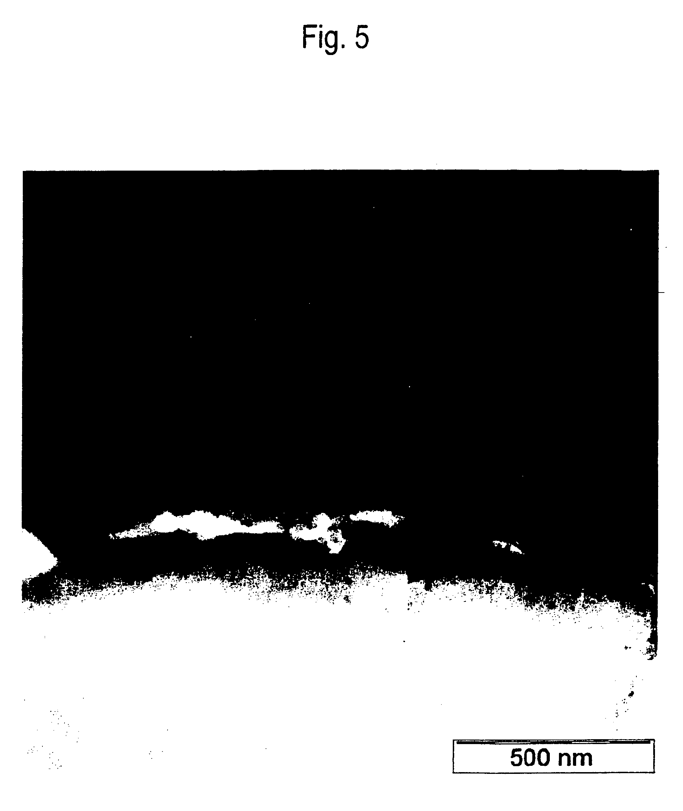

In Figur 5 ist eine TEM-Aufnahme der Beschichtung mit Nanosilber dargestellt. Die typischerweise 20 nm großen Nanosilberpartikel (schwarze Punkte in der TEM Aufnahme) sind nicht aggregiert und sehr gleichmäßig in der Polymerbeschichtung verteilt.

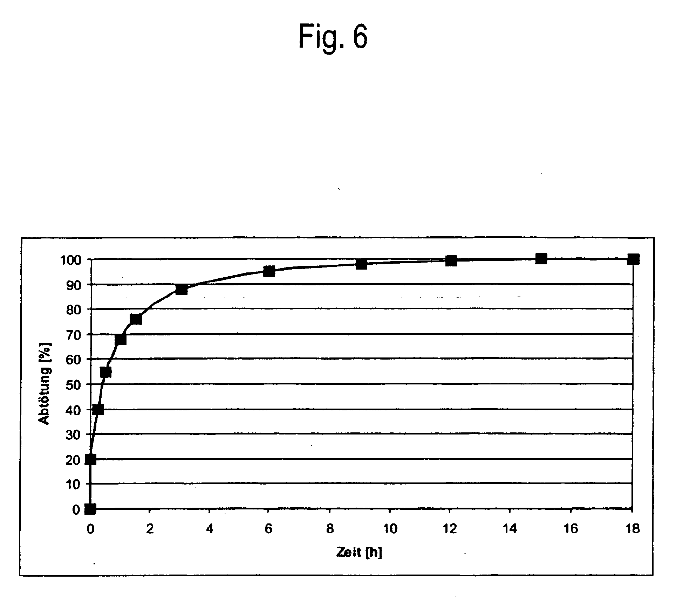

In Figur 6 ist die Kinetik der Abtötung von Bakterien (*E.coli*.), die auf das beschichtete Vlies-/Folienlaminat aufgebracht wurden dargestellt. Bereits nach 3,5 Stunden sind 90 % der aufgebrachten Keime abgetötet.

In der Tabelle 1 ist das Ergebnis eines Mikrobiologischen Tests nach JIS 2801 für das beschriebene Vlies-/Folienlaminat dargestellt. Bei dem Test werden jeweils 2X10⁵ Bakterien auf das mit Nanosilber beschichtete Vlies-/Folienlaminat, auf ein unbeschichtetes Vlies-/Folienlaminat und auf eine Standard Polystyroloberfläche gebracht. Nach einer Kultivierungszeit von 18 Stunden wird die Zahl der lebenden Keime bestimmt. Zusammengefasst lässt sich sagen, dass die Zahl der Keime auf dem Nanosilber beschichteten Vlies-/Folienlaminat im Vergleich zum Standard Polystyrol um 99,8 % reduziert werden, während das unbeschichtete Vlies-/Folienlaminat innerhalb der biologischen Schwankungsbreite keinerlei Keimreduktion aufweist. Damit gilt das Nanosilber beschichtete Vlies-/Folienlaminat als stark antimikrobiell wirksam.

In der Tabelle 2 ist das Ergebnis der Bestimmung der antimycotischen Aktivität des Nanosilber beschichteten Vlies-/Folienlaminats angegeben. Zusammengefasst lässt sich feststellen, dass das Nanosilber beschichtete Vlies-/Folienlaminat eine signifikante Aktivität gegen die 5 getesteten Pilze zeigt.

**Tabelle 1: Mikrobiologischer Test nach JIS 2801 für Nanosilber beschichtetes Vlies-/Folienlaminat mit 150 ppm Silber in der Beschichtung.**

| 1. Test Ergebnis | | | |
|---|---|---|---|
| Nach 0 h | Bakterienzahl Durchschnittswert [cfu] | | |
| Inoculum | 2.1x10⁵ | | |
| Nach 18 h | Bakterienzahl Durchschnittswert [cfu] | | F-Wert |
| interner Standard (Polystyrol) | 2,8x10⁶ | | 1,12 |
| 2-fach Bestimmung unabhängiger Proben | | | |
| Nach 18 h | Bakterienzahl Durchschnittswert [cfu] | % Reduktion ¹ | R-Wert |
| Vlies/Folie Laminat mit Nanosilber Beschichtung | 5,1x10³ | 99,8% | 2,74 |
| Vlies/Folie Laminat ohne Nanosilber Beschichtung | 1,8x10⁶ | 35,6% | 0,19 |
| | | | |
| | | 1) % Reduktion and R-Wert bezogen auf internen Standard | |
| | | | |
| 2. Methode: | JIS Japanese Industrial Standard JIS Z 2801:2000 Antimicrobial products - Test for antimicrobial activity and efficacy. | | |
| | - Plate Count Method - | | |
| | | | |
| Test Bakterien: | *Escherichia Coli* K12 | | |
| | | | |
| Modifikation: | Probengröße:25 mm x 25 mm | | |
| | Berechnung: nur R-Wert | | |
| | Preinkubation C: LB Broth | | |
| | Preinkubation D: LB Broth | | |
| | Inoculations-Medium:1/500 verd. LB Broth (+ 0,13 % Tween 80) | | |
| | Incubation: 37°C | | |
| | Proben Präparation: UVC sterilisiert | | |

**Tabelle 2: Bestimmung der antimycotischen Aktivität für Nanosilber beschichtetes Vlies-/Folienlaminat mit 150 ppm Silber in der Beschichtung.**

| 1. Test Ergebnis | | |
|---|---|---|
| Kontrolle | Pilzwachstum | Kat. |
| Wachstumskontrolle | stark | 6 |
| Kontrobe, Sterilität | nein | |
| Proben | Antimykotische Aktivität | Kat. |
| Baumwolle (interner Standard) | nein | 6 |
| Vlies-Folien-Laminat mit Nanosilber-Beschichtung | signifikant | 3 |
| | | |
| 2. Test Methode | SN 195921 - Textile Fabric - Determination of the Antimycotic Activity - modified | |
| Test Stämme: | DSM 40464 - *Streptomyces abikoensis* | |
| | DSM 9122 - *Scopulariopsis brevicaulis* | |
| | DSM 62413 - *Fusarium solani* | |
| | DSM 10640 - *Penicillium funiculosum* | |
| | DSM 2404 - *Aureobasidium pullulans* | |
| | | |
| Modifikation: | Probengröße: 30 mm Ø | |
| | Preinkubation: Potato Dextrose Agar | |
| | Suspensions-Medium: Potato Dextrose Bouillon | |
| | Proben-Inoculation: Sprühen | |
| | Inkubation: 25°C, Feuchtekammer | |
| | Inkubation-Zeit 40 Tage | |
| | Proben-Sterilisation: UVC | |

### Beispiel 6:

### Verwendung der erfindungsgemäßen Formulierung zur Herstellung von Beschichtungen für textile Dekorstoffe

Die Nanosilber Dispersion aus Beispiel 1 wird in Konzentrationen von 100 mg/kg und 200 mg/kg (bezogen auf den Silbergehalt in der Beschichtung) mit den Mitteln des Standes der Technik in eine handelsübliche, zur Beschichtung von Textilien bestimmte Fluorpolymerdisperion eingearbeitet. Es wird eine stabile, leicht gelb gefärbte Dispersion aus Nanosilber Partikeln und Fluorpolymerpartikeln erhalten. Diese Beschichtung wird auf einen textilen Dekorstoff aufgebracht und thermisch getrocknet/fixiert/vernetzt.

In der Tabelle 3 sind die Ergebnisse mikrobiologischer Tests an Fußmattendekortextilien mit 2 unterschiedlichen hydrophoben, wässrigen Beschichtungen und jeweils zwei Nanosilberdosierungen nach JIS 1902 dargestellt. Die hydrophoben, wässrigen Beschichtungen AG4 und AG8 sind handelsübliche Fluorpolymercoatings. Bei der Beschichtung AG4 wird mittels eines Nanosilber Zusatzes von 100 mg/kg bzw. 200 mg/kg jeweils eine starke Keimreduktion gegenüber einem unbehandelten Textil beobachtet. Bei der Beschichtung AG8 ist der Nanosilber Zusatz von 100 mg/kg zur Hinderung des Keimwachstums nicht ausreichend. Ein Zusatz von 200 mg/kg hingegen bewirkt eine starke Keimreduktion gegenüber einem unbehandelten Textil.

**Tabelle 3: Mikrobiologische Tests nach JIS 1902 an Fußmattendekortextilien mit 2 unterschiedlichen hydrophoben, wässrigen Beschichtungen und jeweils zwei Silberdosierungen.**

| 1. Test Ergebnis | | | |
|---|---|---|---|
| Nach 0h h | Bakterienzahl Durchschnittswert [cfu] | | |
| Inoculum | 1,8x10⁵ | | |
| Nach 18 h | Bakterienzahl Durchschnittswert [cfu] | | F-Wert |
| Faser A0 (Blank) | 1.5x10⁷ | | 1,92 |
| 2-fach Bestimmung unabhängiger Proben | | | |
| Nach 18 h | Bakterienzahl Durchschnittswert [cfu] | % Reduktion ¹ | R-Wert |
| Kontrolle: 080116-xhf01 Mesh fabric (Rita), DTY 160/200F | < 1,0x10² | 99.9993% | 5,18 |
| Fußmattendekormaterial Beschichtung AG4100 ppm | 1,4x10⁴ | 99,9% | 3,03 |
| Fußmattendekormaterial Beschichtung AG4 200 ppm | 2,4x10⁵ | 98,4% | 1,79 |
| Fußmattendekormaterial Beschichtung AG8100 ppm | 4,6x10⁷ | no | -0,49 |
| Pußmattendekormaterial Beschichtung AG8 200 ppm | 7,8x10³ | 99,9% | 3,29 |
| | | | |
| | | 1)% Reduktion and R-Wert bezogen auf Blank | |
| | | | |
| 2. Test Methode: | JIS Japanese Industrial Standard JIS L 1902:2002 "Testing for antibacterial activity and efficacy on textile products". | | |
| | - Plate Count Method - | | |
| | | | |
| Test Stamm: | *Escherichia Coli* K12 | | |
| | | | |
| Modifikation: | Einwaage: 0,4 g | | |
| | Berechnung: nur R-Wert | | |
| | Preinkubation C: LB Broth | | |
| | Preinkubation D: LB Broth | | |
| | Inoculations-Medium: Phosphate Buffered Saline mit 0.05% Tween 80 | | |
| | Inkubation: 37°C | | |

### Beispiel 7:

### Verwendung der erfindungsgemäßen Formulierung zur Herstellung von Synthese Fasern

Die Nanosilber Dispersion aus Beispiel 1 wird in typischen Konzentrationen von 1000 mg/kg bis 20000 mg/kg (bezogen auf den Silbergehalt) mittels Extrusion in handelsübliche Thermoplaste wie z.B. Polypropylen, Polyester, Polyamid eingearbeitet. Dabei werden Masterbatches mit überwiegend isoliert voneinander vorliegenden Nanosilberpartikeln erhalten.

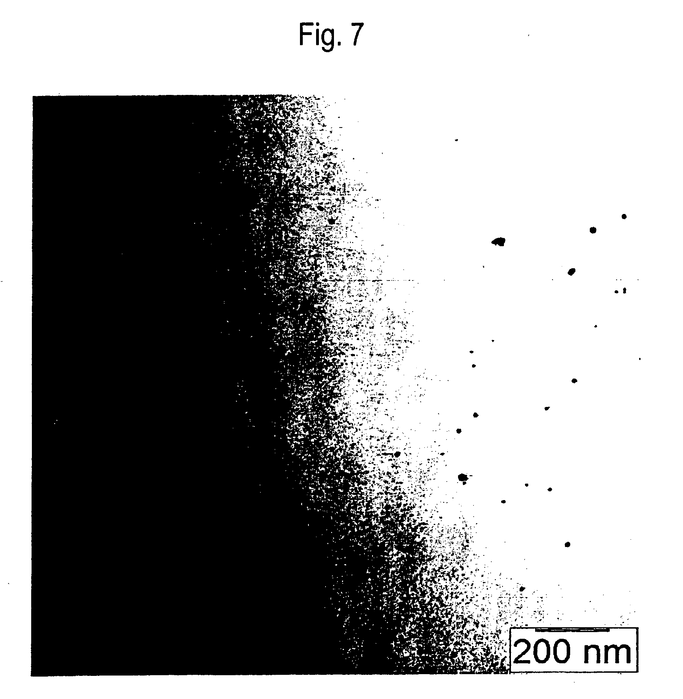

Figur 7 zeigt beispielsweise die TEM-Aufnahme eines Polyester Masterbatches mit 6500 mg/kg Silber. Die dunklen Punkte in Figur 7 zeigen die homogene Verteilung und die geringe Agglomeration der Nanosilberpartikel im Polyester.

Die Masterbatches werden dem Stand der Technik entsprechend verdünnt zur Herstellung von Synthesefasern z.B. aus Polypropylen, Polyester oder Polyamid eingesetzt.

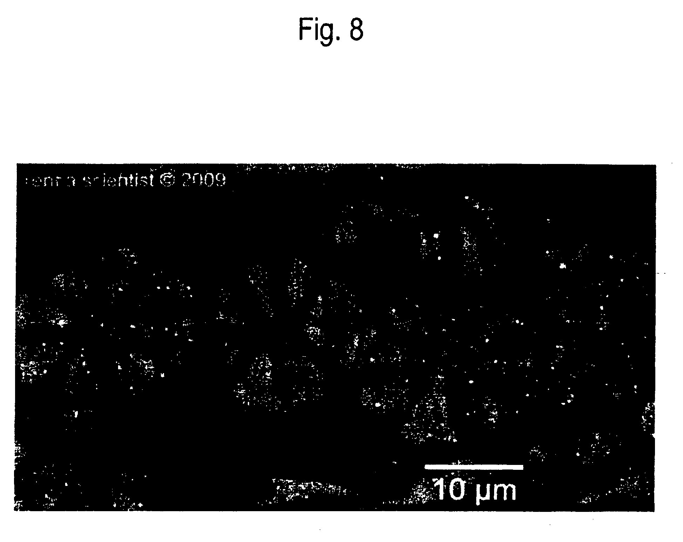

Figur 8 zeigt mehrere Microfaserstränge aus PET/PA mit 200 mg/kg Nanosilber. In der Figur 8 sind die einzelnen Nanosilberpartikel (helle Punkte) in den segmentierten Garnen deutlich zu erkennen. Typische Silbergehalte für antimikrobielle Effekte liegen im Bereich von 100 mg/kg bis 300 mg/kg.

Neben Mikrofasern werden auch Monofil und Bikomponentenfasem für Bekleidung, Bettenfüllungen, Tücher und technische Textilien sowie nonwoven Materialien hergestellt. Die silberhaltigen Synthesefasern können auch in Form von Stapelfasern zur Ausrüstung anderer Fasern (auch Naturfasern wie z.B. Baumwolle) eingesetzt werden. Die eingesetzten Silbergehalte liegen entsprechend der Verdünnung durch andere Fasern auf einem höheren Niveau im Vergleich zur direkten Ausrüstung der Synthesefasern.

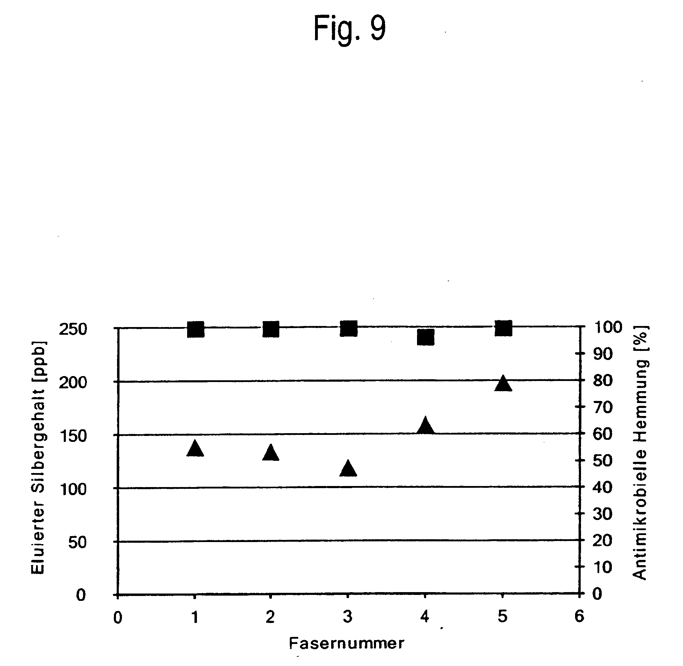

Figur 9 zeigt das Elutionsverhalten (▲) und die antimikrobielle Aktivität (■) verschiedener Polyester Mikrofasern. Der Silbergehalt der verschiedenen Fasern liegt im Bereich von 150 mg/kg bis195 mg/kg. Die Fasern wurden jeweils für 3 h in Wasser eluiert. Der eluierte Silbergehalt liegt im Bereich von 120 µg/kg bis 200 µg/kg Wasser; die antimikrobielle Hemmung liegt jeweils über 96 %. In Tabelle 4 sind die Ergebnisse der antimikrobiellen Tests nach JIS 1902 dieser 5 Mikrofasern dargestellt.

**Tabelle 4: Mikrobiologische Tests nach JIS 1902 an 5 verschiedenen Mikrofasern**

| 1. Test Ergebnis | | | |
|---|---|---|---|
| Nach 0 h | Bakterienzahl Durchschnittswert [cfu] | | |
| Inoculum | 1,4x105 | | |
| Nach 18 h | Bakterienzahl Durchschnittswert [cfu] | | F-Wert |
| Polyesterfaser A0 (Blank) | 2.1x10⁷ | | 2,18 |
| 2-fach Bestimmung unabhängiger Proben | | | |
| Nach 18 h | Bakterienzahl Durchschnittswert [cfu] | % Reduktion¹ | R-Wert |
| Polyesterfaser DTY 160/200F mit 240 ppm Ag | 1.2x10³ | 99,9944% | 4,25 |
| | | | |
| | | 1) % Reduktion und R-Wert bezogen auf Blank | |
| | | | |
| 2. Test Methode: | JIS Japanese Industrial Standard JIS L 1902:2002 *Testing for antibacterial activity and efficacy on textile products". | | |
| | - Plate Count Method - | | |
| | | | |
| Test Stamm: | *Escherichia Coli* K12 | | |
| | | | |
| Modifikation: | Probengewicht: 0,4 g | | |
| | Berechnung: nur R-Wert | | |
| | Preinkubation C: LB Broth | | |
| | Preinkubation D: LB Broth | | |
| | Inoculations-Medium: Phosphate Buffered Saline mit 0.05% Tween 80 | | |
| | Inkubation: 37°C | | |
| | Proben Präparation: nein | | |

### Beispiel 8:

### Herstellung einer stabilen Dispersion von Silbernanopartikel in Acryl- und Epoxidharzen

Für die Herstellung einer Formulierung von Silbernanopartikel in einem Epoxidharz wird das Produkt aus Beispiel 5 verwendet. Dazu werden 992 g Epoxidharz (Araldit™, Huntsman) in einem 2 L Becherglas vorgelegt und auf dem Magnetrührer bei Raumtemperatur gerührt. Nach Zugabe von 8,0 g des Produktes aus Beispiel 5 verändert sich die Farbe der Lösung zu Orangebraun. Durch Schwenken des Glases kann ein dünner Film an der Glaswand generiert werden, der hellgelb, klar gefärbt ist und keine sichtbaren Teilchen enthält. Es werden 1.000 g einer Dispersion erhalten mit einem Silbergehalt von 4.480 mg/kg. Partikelgröße und - verteilung entspricht der Darstellung in den Figuren 2 und 3.

## Patentansprüche

1. Silbernanopartikelhaltige, disperse, wässrige Formulierung umfassend 0,5 bis 60 Gew.-% Silbernanopartikel und zumindest zwei Stabilisatoren aus der Gruppe der nichtionischen Tenside, wobei das Mengenverhältnis Silbernanopartikel zu Stabilisator im Bereich von 10 : 2 bis zu 10 : 50 liegt, wobei die zumindest zwei Stabilisatoren ausgewählt sind aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester, Polyoxypropylen-tri-alkylsäureester und deren Gemische.

2. Formulierung nach Anspruch 1, **dadurch gekennzeichnet, dass** die Stabilisatoren ausgewählt sind aus der Gruppe bestehend aus Polyoxyethylen-Sorbitan-Monolaurat, Polyoxyethylen-Sorbitan-Monopalmitat, Polyoxyethylen-Sorbitan-Monostearat, Polyoxyethylen-Sorbitan-Monooleat, Polyoxyethylen-Sorbitan-Tristearat, Polyoxyethylen-Glyceryl-Trioleat, Polyoxyethylen-Glyceryl-Monolaurat, Polyoxyethylen-Glyceryl-Monooleat, Polyoxyethylen-Glyceryl-Monostearat, Polyoxyethylen-Glyceryl-Monoricinoleat, Rizinusöl, hydriertes Rizinusöl, Sojabohnenöl und deren Gemische.

3. Formulierung nach einem der Ansprüche 1 und 2, **dadurch gekennzeichnet, dass** die Stabilisatoren in einem Mengenverhältnis im Bereich von 1 : 1 bis 2 : 1 anwesend sind.

4. Formulierung nach einem der Ansprüche 1 bis 3, **dadurch gekennzeichnet, dass** die Silbernanopartikel in einer Partikelgröße von 1 nm bis 50 nm vorliegen.

5. Formulierung nach einem der Ansprüche 1 bis 4, **dadurch gekennzeichnet, dass** die Silbernanopartikel in einer Partikelgröße von 1 nm bis 20 nm vorliegen.

6. Formulierung nach einem der Ansprüche 1 bis 5, **dadurch gekennzeichnet, dass** das Mengenverhältnis Silbernanopartikel zu Stabilisator im Bereich von 10 : 5 bis zu 10: 10 liegt.

7. Formulierung nach Anspruch 6, **dadurch gekennzeichnet, dass** die Silbernanopartikel in einem Anteil von 1 Gew.-% bis 40 Gew.-% in der Formulierung enthalten sind.

8. Formulierung nach einem der Ansprüche 1 bis 7, **dadurch gekennzeichnet, dass** die Formulierung zumindest 70 Gew.-% Wasser enthält.

9. Verfahren zur Herstellung einer silbernanopartikelhaltigen, dispersen, wässrigen Formulierung gemäß den Ansprüchen 1 bis 8 umfassend die Schritte
- Bereitstellen eines Silbersalzes,
- Bereitstellen von zumindest zwei Stabilisatoren ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester und Polyoxypropylen-tri-alkylsäureester und der Gemischen,
- Bereitstellen eines Reduktionsmittels,
- Bereitstellen des Lösungsmittels Wasser,
- Herstellen einer Lösung von Silbersalz, Stabilisator und Reduktionsmittel,
- Zugabe einer Base zu der Lösung, wobei die Zugabe der Base kontinuierlich über einen Zeitraum von 5 h bis 48 h derart erfolgt, dass der pH-Wert der Formulierung zwischen 0 und 6 liegt.

10. Verfahren nach Anspruch 9, **dadurch gekennzeichnet, dass** das Reduktionsmittel mit den Silberionen des Silbersalzes unter Bildung von elementarem Silber und ansonsten überwiegend gasförmigen Reaktionsprodukten reagiert.

11. Verfahren nach einem der Ansprüche 9 und 10, **dadurch gekennzeichnet, dass** als Reduktionsmittel Hydrazinhydrat eingesetzt wird.

12. Verfahren nach einem der Ansprüche 9 bis 11, **dadurch gekennzeichnet, dass** als Base eine Base mit einem pK_{b} Wert im Bereich von -2 bis 10,5 eingesetzt wird.

13. Verfahren nach einem der Ansprüche 9 bis 12, **dadurch gekennzeichnet, dass** als Base Ammoniak, Kaliumhydrogencarbonat oder Natriumhydroxid eingesetzt wird.

14. Verfahren nach einem der Ansprüche 9 bis 13, **dadurch gekennzeichnet, dass** die Zugabe der Base kontinuierlich über einen Zeitraum von 9 h bis 30 h erfolgt.

15. Verwendung einer Formulierung nach einem der Ansprüche 1 bis 8 oder einer nach einem Verfahren nach einem der Ansprüche 9 bis 14 hergestellten Formulierung zur Oberflächenbehandlung von Holz und/oder in Imprägnierölen, für Luftfilter und/oder als textile Färbelösung und/oder zur Förderung des Pflanzenwachstums und/oder zur Herstellung antimikrobieller Oberflächen und/oder zur Erzielung elektrischer Leitfähigkeit und/oder zur Erzielung antistatischer Eigenschaften und/oder als Katalysator und/oder zur Beschichtung von Vlies-/Folienlaminaten und/oder zur Beschichtung von textilen Dekorstoffen und/oder in Lacken und Klebstoffen und/oder in Silikonen und/oder in thermoplastischen Kunststoffen und/oder in Wood Plastic Composites (WPC) und/oder in Polyolefin Formkörpern und/oder in PVC und/oder Plastisolen und/oder zur Herstellung von PVC Plastisol beschichteten Textilien und/oder in Duroplasten und/oder zur Herstellung von Fasern, insbesondere zur Herstellung von Synthesefasern und Regeneratfasern.

## Claims

1. A dispersed aqueous formulation containing silver nanoparticles, comprising 0.5% to 60% by weight of silver nanoparticles and at least two stabilizers from the non-ionic surfactant group, wherein the proportion of silver nanoparticles to stabilizer is in the range of 10:2 to 10:50, wherein the at least two stabilizers are selected from the group consisting of polyoxyethylene-mono-alkyl acid esters, polyoxypropylene-mono-alkyl acid esters, polyoxyethylene-di-alkyl acid esters, polyoxypropylene-di-alkyl acid esters, polyoxyethylene-tri-alkyl acid esters, polyoxypropylene-tri-alkyl acid esters and mixtures thereof.

2. The formulation according to claim 1, **characterized in that** the stabilizers are selected from the group consisting of polyoxyethylene-sorbitan-monolaurate, polyoxyethylene-sorbitan-monopalmitate, polyoxyethylene-sorbitan-monostearate, polyoxyethylene-sorbitan-monooleate, polyoxyethylene-sorbitan-tristearate, polyoxyethylene-glyceryl-trioleate, polyoxyethylene-glyceryl-monolaurate, polyoxyethylene-glyceryl-monooleate, polyoxyethylene-glyceryl-monostearate, polyoxyethylene-glyceryl-monoricinoleate, castor oil, hydrated castor oil, soyabean oil and mixtures thereof.

3. The formulation according to one of claims 1 and 2, **characterized in that** the stabilizers are present in a proportion in the range 1:1 to 2:1.

4. The formulation according to one of claims 1 to 3, **characterized in that** the silver nanoparticles are present in a particle size of 1 nm to 50 nm.

5. The formulation according to one of claims 1 to 4, **characterized in that** the silver nanoparticles are present in a particle size of 1 nm to 20 nm.

6. The formulation according to one of claims 1 to 5, **characterized in that** the proportion of silver nanoparticles to stabilizer is in the range of 10:5 to 10:10.

7. The formulation according to claim 6, **characterized in that** the percentage of silver nanoparticles contained in the formulation is 1% by weight to 40% by weight.

8. The formulation according to one of claims 1 to 7, **characterized in that** the formulation contains at least 70% by weight of water.

9. A method for producing a disperse aqueous formulation containing silver nanoparticles as claimed in claims 1 to 8, comprising the following steps:
- providing a silver salt,
- providing at least two stabilizers selected from the group consisting of polyoxyethylene-mono-alkyl acid esters, polyoxypropylene-mono-alkyl acid esters, polyoxyethylene-di-alkyl acid esters, polyoxypropylene-di-alkyl acid esters, polyoxyethylene-tri-alkyl acid esters, polyoxypropylene-tri-alkyl acid esters and mixtures thereof,
- providing a reducing agent,
- providing the solvent water,
- producing a solution of silver salt, stabilizer and reducing agent,
- adding a base to the solution whereby the base is added continuously over a period of 5 h to 48 h in a manner such that the pH of the formulation is between 0 and 6.

10. The method according to claim 9, **characterized in that** the reducing agent reacts with the silver ions of the silver salt by forming elemental silver and otherwise, substantially gaseous reaction products.

11. The method according to one of claims 9 and 10, **characterized in that** hydrazine hydrate is used as the reducing agent.

12. The method according to one of claims 9 to 11, **characterized in that** a base with a pK_{b} in the range of -2 to 10.5 is used as the base.

13. The method according to one of claims 9 to 12, **characterized in that** ammonia, potassium bicarbonate or sodium hydroxide is used as the base.

14. The method according to one of claims 9 to 13, **characterized in that** the base is added continuously over a period of 9 h to 30 h.

15. Use of a formulation as claimed in one of claims 1 to 8 or a formulation produced by means of a method as claimed in one of claims 9 to 14, for the surface treatment of wood and/or in impregnating oils for air filters and/or as a textile staining solution and/or for promoting the growth of plants and/or for the production of antimicrobial surfaces and/or for producing electrical conductivity and/or for producing antistatic properties and/or as a catalyst and/or for coating nonwoven/foil laminates and/or for coating decorative textiles and/or in paints and adhesives and/or in silicones and/or in thermoplastic synthetics and/or in wood plastic composites (WPC) and/or in polyolefin mouldings and/or in PVC and/or in plastisols and/or for the production of textiles coated with PVC plastisol and/or in duroplasts and/or for the production of fibres, in particular for the production of synthetic fibres and regenerated fibres.

## Revendications

1. Formulation aqueuse en dispersion contenant des nanoparticules d'argent, comprenant de 0,5 à 60 % en poids de nanoparticules d'argent et au moins deux stabilisateurs du groupe des tensioactifs non ioniques, la proportion des nanoparticules d'argent par rapport au stabilisateur se situant dans l'ordre de 10 : 2 à 10 : 50, les au moins deux stabilisateurs étant choisis dans le groupe constitué de l'ester d'acide polyoxyéthylène-mono-alkyle, l'ester d'acide polyoxypropylène-mono-alkyle, l'ester d'acide polyoxyéthylène-di-alkyle, l'ester d'acide polyoxypropylène-di-alkyle, l'ester d'acide polyoxyéthylène-tri-alkyle, l'ester d'acide polyoxypropylène-tri-alkyle et de leurs mélanges.

2. Formulation selon la revendication 1, **caractérisée en ce que** les stabilisateurs sont choisis dans le groupe constitué du monolaurate de polyoxyéthylène sorbitane, du monopalmitate de polyoxyéthylène sorbitane, du monostéarate de polyoxyéthylène sorbitane, du monooléate de polyoxyéthylène sorbitane, du tristéarate de polyoxyéthylène sorbitane, du trioléate de polyoxyéthylène glycéryle, du monolaurate de polyoxyéthylène glycéryle, du monooléate de polyoxyéthylène glycéryle, du monostéarate de polyoxyéthylène glycéryle, du monoricinoléate de polyéthylène glycéryle, de l'huile de ricin, de l'huile de ricin hydrogénée, de l'huile de soja et de leurs mélanges.

3. Formulation selon l'une quelconque des revendications 1 et 2, **caractérisée en ce que** les stabilisateurs sont présents dans une proportion de l'ordre de 1 : 1 à 2:1.

4. Formulation selon l'une quelconque des revendications 1 à 3, **caractérisée en ce que** les nanoparticules d'argent sont présentes avec une taille de particules de 1 nm à 50 nm.

5. Formulation selon l'une quelconque des revendications 1 à 4, **caractérisée en ce que** les nanoparticules d'argent sont présentes avec une taille de particules de 1 nm à 20 nm.

6. Formulation selon l'une quelconque des revendications 1 à 5, **caractérisée en ce que** la proportion de nanoparticules d'argent en rapport au stabilisateur se situe dans l'ordre de 10 : 5 à 10 : 10.

7. Formulation selon la revendication 6, **caractérisée en ce que** les nanoparticules d'argent sont contenues dans la formulation dans une part de 1 % en poids à 40 % en poids.

8. Formulation selon l'une quelconque des revendications 1 à 7, **caractérisée en ce que** la formulation contient au moins 70 % en poids d'eau.

9. Procédé destiné à la préparation d'une formulation aqueuse en dispersion contenant des nanoparticules d'argent selon les revendications 1 à 8, comprenant les étapes :
- Mise à disposition d'un sel d'argent,
- Mise à disposition d'au moins deux stabilisateurs choisis dans le groupe contenant l'ester d'acide polyoxyéthylène-mono-alkyle, l'ester d'acide polyoxypropylène-mono-alkyle, l'ester d'acide polyoxyéthylène-di-alkyle, l'ester d'acide polyoxypropylène-di-alkyle, l'ester d'acide polyoxyéthylène-tri-alkyle, l'ester d'acide polyoxypropylène-tri-alkyle et de leurs mélanges,
- Mise à disposition d'un agent réducteur,
- Mise à disposition du solvant eau,
- Préparation d'une solution de sel d'argent, de stabilisateur et d'agent réducteur,
- Ajout d'une base à la solution, l'ajout de la base s'effectuant en continu sur une période de 5 h à 48 h, de sorte que la valeur pH de la formulation se situe entre 0 et 6.

10. Procédé selon la revendication 9, **caractérisé en ce que** l'agent réducteur réagit avec les ions d'argent du sel d'argent en formant de l'argent élémentaire et par ailleurs principalement des produits de réaction gazeux.

11. Procédé selon l'une quelconque des revendications 9 et 10, **caractérisé en ce qu'**on met en oeuvre de l'hydrate d'hydrazine en tant qu'agent réducteur.

12. Procédé selon l'une quelconque des revendications 9 à 11, **caractérisé en ce qu'**en tant que base, on met en oeuvre une base avec une valeur pK_{b} de l'ordre de -2 à 10,5.

13. Procédé selon l'une quelconque des revendications 9 à 12, **caractérisé en ce qu'**en tant que base, on met en oeuvre de l'ammoniac, de l'hydrogénocarbonate de potassium ou de l'hydroxyde de sodium.

14. Procédé selon l'une quelconque des revendications 9 à 13, **caractérisé en ce que** l'ajout de la base s'effectue en continu sur une période de 9 h à 30 h.

15. Utilisation d'une formulation selon l'une quelconque des revendications 1 à 8 ou d'une formulation préparée selon l'une quelconque des revendications 9 à 14 pour le traitement de surfaces de bois et/ou dans des huiles d'imprégnation pour des filtres à air et/ou en tant que solution de teinture textile et/ou pour stimuler la croissance de plantes et/ou pour fabriquer des surfaces antimicrobiennes et/ou pour obtenir une conductibilité électrique et/ou pour obtenir des caractéristiques antistatiques et/ou en tant que catalyseur et/ou pour revêtir des stratifiés de film/de non-tissé et/ou pour le revêtement de matières textiles décoratives et/ou dans des laques et des agents adhésifs et/ou dans des silicones et/ou dans des matières thermoplastiques et/ou dans des bois composites (Wood Plastic Composites WPC) et/ou dans des PVC et/ou dans des plastisols et/ou pour la fabrication de textiles revêtus de PVC plastisol et/ou dans des résines thermodurcissables et/ou pour la fabrication de fibres, notamment pour la fabrication de fibres synthétiques et de fibres régénérées.
